# EUROPÄISCHE PATENTSCHRIFT

(11) **EP 2 481 293 B1**
(45) Veröffentlichungstag und Bekanntmachung des Hinweises auf die Patenterteilung: **30.04.2014**
(21) Anmeldenummer: 11152404.7
(22) Anmeldetag: 27.01.2011
(51) Int. Cl.: A23J 1/00, A23K 1/06, C12F 3/10, A23K 1/16

(54) **Verfahren zur Verarbeitung von Dünnschlempe und Vorrichtung zur Herstellung eines proteinhaltigen Produktes**
Method for processing distillery spent grains and device for producing a protein containing product
Procédé de traitement de résidus solubles de distillation et dispositif de fabrication d'un produit riche en protéines

(43) Veröffentlichungstag der Anmeldung: 01.08.2012
(73) Patentinhaber: GEA Mechanical Equipment GmbH, 59302 Oelde (DE)
(72) Erfinder: Emanuele, Mario, 59302, Oelde (DE); Schöneberg, Knud, 21684, Stade (DE)
(74) Vertreter: Specht, Peter

(56) Entgegenhaltungen:
- EP-A2- 0 237 520
- DE-A1- 3 805 398
- RICHARD P. EGG ET AL: "Grain sorghum stillage recycling: Effect on ethanol yield and stillage quality", BIOTECHNOLOGY AND BIOENGINEERING, Bd. 27, Nr. 12, 1. Dezember 1985 (1985-12-01), Seiten 1735-1738, XP55001988, ISSN: 0006-3592, DOI: 10.1002/bit.260271217

## Beschreibung

Die Erfindung betrifft ein Verfahren nach dem Oberbegriff des Anspruchs 1 und eine Vorrichtung nach dem Oberbegriff des Anspruchs 10.

Bei der Gewinnung von Ethanol im Destillationsverfahren aus Getreide, wie Mais, Weizen und/oder Triticale fällt ein großer Anteil an Schlempe an.

Aus dieser Schlempe kann u.a. Futter für die Tierhaltung gewonnen werden.

Ein Verfahren zum Verarbeiten von Dünnschlempe u.a. mit einem Hydrozyklon offenbart die DE OS 38 05 398.

In einem anderen, bislang üblichen Verfahren wird die Schlempe mittels eines Dekanters in Dünn- und Dickschlempe getrennt. Die Dünnschlempe wird einem Verdampfer zugeführt, um den Wassergehalt weiter abzusenken und eine weitere Aufkonzentrierung zu erreichen, wobei man nach diesem Verfahrensschritt vom sogenannten Schlempesirup spricht. Anschließend wird die Dickschlempe wieder mit dem Schlempesirup vermischt und einem Trockner zugeführt, um einen Trockensubstanzgehalt von über 90 Gew.-% zu erreichen. Dieses gewonnene Futter kann allerdings nur an Wiederkäuer verfüttert werden, da der Anteil an faserhaltigem Material sehr hoch ist und die Verwendung dieses nahrhaften Futterzusatzes daher auf einige Anwendungsbereiche beschränkt ist.

Die DE 20 2009 013 389.3 offenbart eine Vorrichtung zur Gewinnung eines Produktes bei dem die Dünnschlempe durch Ultrafiltration aufkonzentriert und anschließend zusammen mit der Dickschlempe in einer Trocknungsvorrichtung zusammengeführt wird. Diese Vorrichtung hat sich grundsätzlich bewährt, allerdings kann auch in diesem Fall das hergestellte Produkt nur an Wiederkäuer verfüttert werden. Zudem hat sich gezeigt, dass die Ultrafiltration bei der Verarbeitung von Dünnschlempe nur bis zu einer bestimmten Konzentrierungsgrad wirtschaftlich betrieben werden kann.

Es ist nunmehr Aufgabe der vorliegenden Erfindung einerseits ein Verfahren bereitzustellen, welches eine optimale Betriebsweise einer Filtrationsanlage gewährleistet und andererseits eine Vorrichtung zu schaffen, um ein protein- und fetthaltiges, im Wesentlichen faserfreies, Wertprodukt herzustellen.

Die Erfindung löst diese Aufgaben durch ein Verfahren mit den Merkmalen des Anspruchs 1 und eine Vorrichtung mit den Merkmalen des Anspruchs 10.
Im Anschluss an das Vorkonzentrieren der Dünnschlempe durch die Filtrationsanlage erfolgt eine Strömungsteilung, wobei ein erster Teilstrom in den Arbeitsbehälter rückgeführt wird und ein zweiter Teilstrom weiterverarbeitet wird.

Durch das Rückführen des ersten Teilstromes an vorkonzentrierter Dünnschlempe zu der in dem Arbeitsbehälter befindlichen Dünnschlempe kann der Trockensubstanzgehalt dieser Dünnschlempe erhöht werden, was eine optimalere Arbeitsweise und eine höhere Effizienz der Filtrationsanlage ermöglicht.

Vorteilhafte Ausgestaltungsvarianten der Erfindung sind Gegenstand der Unteransprüche.

Dieser zweite Teilstrom kann im Anschluss von der Filtrationsanlage an ein mechanisches fest-flüssig Trenngerät weitergeleitet werden, in welchem die Dünnschlempe nochmals auf konzentriert wird, so dass vorteilhaft eine Dünnschlempe mit einem Trockensubstanzgehalt von vorzugsweise größer als 12 Gew.-% gebildet wird. Durch die Kombination einer Filtrationsanlage mit einer Zentrifuge kann vorteilhaft ein besonders temperaturschonendes Aufkonzentrieren der Dünnschlempe erfolgen.

Nach der weiteren Aufkonzentrierung der Dünnschlempe durch die Klärung und Abführung der geklärten wässrigen Phase wird die abgeschleuderte Dünnschlempe als Düsenphase des Separators in eine Trocknungsvorrichtung, beispielsweise einem Sprühtrockner, überführt, wo sich durch Trocknen der konzentrierten Dünnschlempe ein proteinhaltiges im Wesentlichen faserfreies Wertproduktes bildet, welches beispielsweise als Futterzusatz geeignet ist.

Die Trocknung kann produktschonend erfolgen, also keine Trocknung in Öfen, Flammen oder dergleichen, so dass wertvolle Inhaltsstoffe durch Wärmeeinwirkung nicht geschädigt werden. Die Trocknung kann allerdings auch nach der Bearbeitung in der Zentrifuge entfallen.

Die dem Arbeitsbehälter zugeführte Dünnschlempe kann vorteilhaft durch Verarbeitung von Getreideschlempe, beispielsweise im Anschluss an eine Destillation, in einem Dekanter bereitgestellt werden.

Es ist weiterhin von Vorteil, wenn zusätzlich zur aufkonzentrierten Dünnschlempe ein Permeatstrom aus der Filtrationsanlage abgeführt wird, welcher einer z.B anaeroben Abwasserreinigung zuführbar ist oder wieder als Prozesswasser verwendet wird. Dies ist von Vorteil, da dadurch das Abwasser problemlos aufgearbeitet werden kann und keiner gesonderten Sammelung und Lagerung, wie es häufig bei Prozessabwässern auftritt in diesem Fall vermieden werden kann.

Erfindungsgemäß weist eine Vorrichtung zur Herstellung eines proteinhaltigen Wertproduktes aus Dünnschlempe, einem Arbeitsbehälter, einer Filtrationsanlage, einer Zentrifuge, vorzugsweise einem Separator, und eine Trocknungsvorrichtung auf, wobei die Filtrationsanlage mit dem Arbeitsbehälter über eine Rückführung für einen ersten Teilstrom der konzentrierten Dünnschlempe verbunden ist. Durch diese Arbeitsweise wird ein nahezu faserfreies Wertprodukt geschaffen, wobei die Filtrationsanlage betriebseffizient arbeitet.

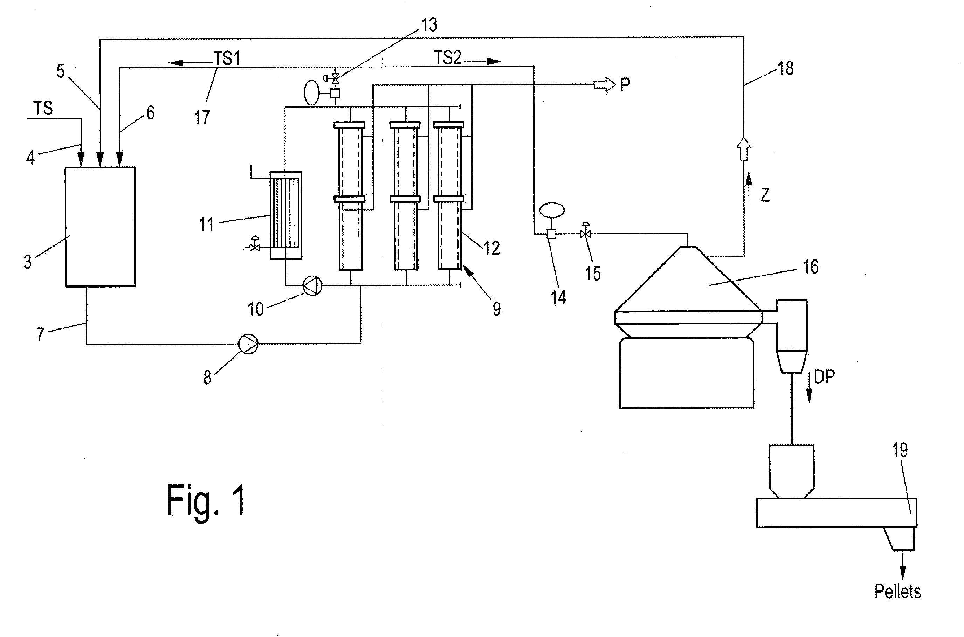

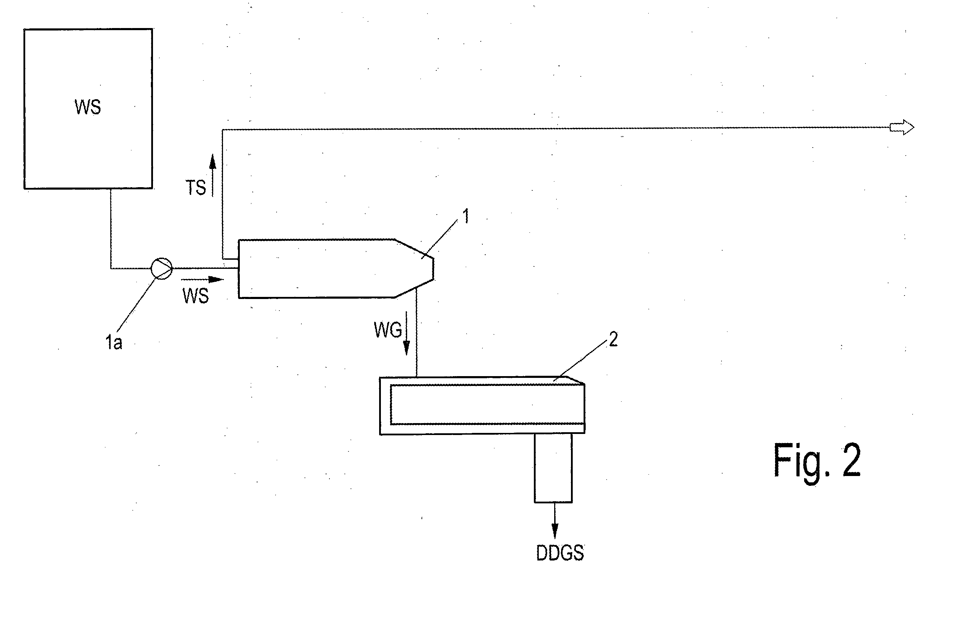

Die Erfindung wird nachfolgend anhand eines Ausführungsbeispiels mit Hilfe der Zeichnungen Fig.1 und 2 näher erläutert. Sie zeigen:
- Fig.1: Eine schematische Darstellung eines an sich bekannten Verfahrens zur Bereitstellung von Dünnschlempe und
- Fig.2: Eine schematische Darstellung einer Vorrichtung zur Herstellung eines Wertproduktes aus Dünnschlempe.

Fig.1 zeigt an sich bekannte Verarbeitungsschritte einer Getreideschlempe (whole stillage) WS, beispielsweise im Anschluss an eine Ethanoldestillation. Diese Schlempe WS weist einen Trockensubstanzgehalt von etwa 10 Gew.-% auf, wobei etwa 7 Gew.-% auf suspendierte Feststoffe und 3 Gew.-% auf gelöste Feststoffe entfallen und wird über eine Pumpe 1 a in einen Dekanter 1 eingeleitet, wo sie zu Dickschlempe (wet grains) WG und Dünnschlempe (thin stillage) TS verarbeitet wird.

Der Anteil an Trockensubstanz in der Dünnschlempe TS beträgt dabei etwa 4,15 Gew.-%, bezogen auf die Gesamtmasse der Dünnschlempe TS, wobei etwa 1,15 Gew.-% auf suspendierte Feststoffe entfallen und etwa 3 Gew.-% auf gelöste Feststoffe. Der Anteil an Trockensubstanz in der Dickschlempe WG ist größer als 30 Gew.-%, bezogen auf die Gesamtmasse der Dickschlempe WG.

Vom Dekanter 1 wird die Dickschlempe in eine Trocknungsvorrichtung, z.B. einen Trommeltrockner 2 geleitet, wo die Trägerflüssigkeit der Dickschlempe, meist Wasser, weitestgehend verdampft wird, so dass sich ein nährstoffreicher Feststoff, die sog. Trockenschlempe (Dried Destillers Grains with Solubles) DDGS, bildet, welcher u.a. als Futtermittel für die Tierhaltung eingesetzt wird.

Die Dünnschlempe weist einen Volumenstrom von 6-7 m³/h auf. Und wird im Anschluss an einen Arbeitsbehälter 3 weitergeleitet.

Die Gewinnung von Proteinen und Fetten aus Dünnschlempe TS wird durch ein konkretes Ausführungsbeispiel anhand der Anlage in Fig.2 näher beschrieben.

Der Arbeitsbehälter 3 fasst im konkreten Ausführungsbeispiel 20m³ und weist insgesamt drei Zuläufe 4, 5 und 6 auf. Der Zulauf 4 des Arbeitsbehälters 3 ist ein Zulauf zum Zuführen von Dünnschlempe (TS) in die Anlage, welche beispielsweise vom Dekanter 1 in Fig.1 zugeführt wird. Die weiteren Zuläufe 5 und 6 zum Arbeitsbehälter 3 sind für aus dem Prozess rückgeführte Dünnschlempe und für Fugatphase vorgesehen.

Der Arbeitsbehälter 3 weist zudem einen Ablauf 7 auf, welcher den Arbeitsbehälter 3 mit einer Ultrafiltrationsanlage 9 verbindet und dient zur Überleitung der Dünnschlempe aus dem Arbeitsbehälter 3 in die Ultrafiltrationsanlage 9. Der Transport der Dünnschlempe vom Arbeitsbehälter 3 zur Ultrafiltrationsanlage 9 erfolgt mit Hilfe einer Speisepumpe 8 unter einem Druck von 3-4 bar wobei die Speisepumpe eine Leistung von vorzugsweise 15 KW erbringt.

Die Ultrafiltrationsanlage 9 ist in Fig.2 lediglich schematisch dargestellt und weist zumindest eine Loop-pumpe 10, einen Kühler 11 und ein oder mehrere Ultrafiltrationsmodule 12 auf.

Während der Filtration wird Flüssigkeit aus der Dünnschlempe abgezogen und als Permeat P mit einem Volumenstrom von etwa 6 m³/h aus der Ultrafiltrationsanlage 9 abgeführt.

In den Ultrafiltrationsmodule 12 ist, hier nicht dargestellt, eine Anzahl an keramischen Membranfilterstäben vorzugsweise aus alpha-Aluminiumoxid und Zirconiumoxid angeordnet. Diese Membranfilterstäbe sind in Längsrichtung mit parallelverlaufenden Kanälen durchsetzt, wobei die Kanäle derart ausgestattet sind, dass sie entlang der Kanalwandung vollumfänglich eine Filtermembran, bestehend aus Zirconiumoxid aufweisen, welches auf eine Trägersubstanz, bestehend aus alpha-Aluminiumoxid aufgebracht ist. Dabei weist die Zirconiumoxidschicht fein verteilte Poren auf, während das Trägermaterial aus Aluminiumoxid grobporig aufgebaut ist. Die Filtermembran weist dabei eine durchschnittliche Porengröße von 50nm auf.

In den in Fig.2 dargestellten sechs Ultrafiltrationsmodulen sind insgesamt 168 Membranfilterstäbe angeordnet, die eine Filterfläche von 72 m² zur Abtrennung von Flüssigkeit aus der Dünnschlempe bereitstellen. Dabei wird das Permeat mit den durch die Speisepumpe erzeugten 3-4 bar durch die keramische Filtermembran gedrückt. Über ein Regelventil Ventil 13 wird das Retentat bzw. die vorkonzentrierte Dünnschlempe aus der Ultrafiltrationsanlage abgeführt.

Während der Filtration lagern sich Feststoffe, die nicht durch die 50 nm großen Poren der Filtermembran gelangen, auf der Oberfläche der Membran an. Um ein Verstopfen der Filtermembran zu verhindern, kann durch die Loop-pumpe 10, bei geschlossenem Ventil 13, eine turbulente Strömung von 5m/s bei einem Volumenstrom von 810 m³/h erzeugt werden. Bei einer derartigen Strömung werden die angelagerten Feststoffpartikel von der Filtermembran gelöst bzw. durch die Strömung abgetragen.

Nach der Filtration verlässt eine aufkonzentrierte Dünnschlempe als Retentat die Ultrafiltrationsanlage und wird in zwei Teilströme TS1 und TS2 aufgeteilt.

Ein zweiter Teilstrom an Retentat bzw. aufkonzentrierter Dünnschlempe TS2, der vorzugsweise etwa 4-5 m³/h beträgt, wird einer Zentrifuge, vorzugsweise einem Zweiphasen-Separator 16, zugeführt. Der Umfang des Teilstromes kann durch Mittel zur Strömungsteilung, hier ein Ventil 15 beispielsweise in Abhängigkeit von der Durchflussrate geregelt werden. Diese Durchflussrate kann durch einen Sensor 14 ermittelt werden, welcher anschließend über das Ventil 15 den Durchfluss an einen vorgegebenen Sollwert anpasst.

In der Zentrifuge wird das in der Ultrafiltration erzeugte Retentat auf einen Trockensubstanzgehalt von vorzugsweise größer als 12 Gew.-% aufkonzentriert, wobei am Oberlauf der Zentrifuge eine geklärte Flüssigkeit als Fugat Z abgeführt wird

Das Fugat Z weist einen Feststoffanteil von weniger als einen Vol.-% auf und wird mit einem Volumenstrom von 2,2-3,2 m³/h über den Rücklauf 18 und den Zulauf 5 von der Zentrifuge 16 in den Arbeitsbehälter rückgeführt. Die Düsenphase DP bzw. die abgeschleuderte Phase weist einen Trockensubstanzgehalt von mehr als größer als 12 Gew.-%, vorzugsweise bis zu 18 Gew.-% und einem FC-Faktor von 7-8 auf, und wird anschließend mit eine hier nicht dargestellte Trocknungsvorrichtung zu einem transport- und lagerfähigen Produkt verarbeitet. Dieses Produkt kann beispielsweise durch eine Pelletierpresse 19 in eine kompaktere Form, zu Pellets, umgewandelt werden. Die so hergestellten Pellets können als wertvoller Futterzusatz verwendet werden und weist einen Anteil an Trockensubstanz von über 90Gew.-% auf, wovon 40% Proteine, 55% Fett und ein Restanteil auf Fasern und Asche entfällt.

Ein anteiliger zweiter Volumenteilstrom TS2 von 30m³/h wird mittels der Durchflussregelung über eine Rückführung 17 und den Zulauf 6 zum Arbeitsbehälter 3 rückgeführt und der Dünnschlempe vom Dekanter aus dem Zulauf 4 vermischt, so dass sich ein Trockensubstanzgehalt für die Dünnschlempe im Arbeitsbehälter 3 von etwa 7 Gew.-% einstellt und der FC-Faktor, einem Konzentrationsfaktor, um den Faktor 3 bis 4, gegenüber der Dünnschlempe aus dem Dekanter 1, erhöht wird.

Durch die Rückführung 17 zum Rückführen bzw. Rückleiten eines zweiten Teilstroms TS2 an Retentat in den Arbeitsbehälter 3 erfolgt eine Aufkonzentrierung der aus dem Dekanter 1 abgeführten Dünnschlempe TS. Es hat sich überraschend gezeigt, dass durch die Aufkonzentrierung der Dünnschlempe TS auf einen Trockensubstanzgehalt von etwa 7% der optimale Betriebspunkt für eine Ultrafiltrationsanlage 9 bei der Verarbeitung von Dünnschlempe erreicht wird.

Aus dem aus der Ultrafiltrationsanlage 9 abgetrennten Permeat P, das einen Umfang von vorzugsweise 6 m³/h umfasst und hauptsächlich Wasser aufweist, wurden Feststoffpartikel im Größenbereich 5-100nm entfernt, so dass dieses Permeat der kommunalen Abwasserreinigung zuführbar ist oder einer anaeroben Abwasserreinigung unterzogen werden kann. Des weiteren könnte die wässrige Phase als Prozesswasser beispielsweise zur Anmeischung weiter genutzt werden.

## Patentansprüche

1. Verfahren zur Verarbeitung von Dünnschlempe (TS), **gekennzeichnet durch** folgende Schritte:
a) Zuführen von Dünnschlempe (TS) in einen Arbeitsbehälter (3);
b) Aufkonzentrieren von Dünnschlempe (TS) in einer Filtrationsanlage (9);
c) wobei nach dem Aufkonzentrieren von Dünnschlempe in der Filtrationsanlage (9) eine Strömungsteilung der aufkonzentrierten Dünnschlempe in zumindest den ersten Teilstrom (TS1) und einen zweiten Teilstrom (TS2) erfolgt, und
d) Rückführen des ersten Teilstromes (TS1) an aufkonzentrierter Dünnschlempe zu der in den Arbeitsbehälter (3) befindlichen Dünnschlempe (TS) zur Einstellung des Gehaltes an Trockensubstanz.

2. Verfahren nach Anspruch 1, **dadurch gekennzeichnet, dass** nach der Strömungsteilung eine Klärung zumindest des zweiten Teilstromes (TS2) der aufkonzentrierten Dünnschlempe durch eine Zentrifuge, vorzugsweise durch einen Separator (16) erfolgt, wobei ein weiteres Aufkonzentrieren der Dünnschlempe erfolgt.

3. Verfahren nach Anspruch 2, **dadurch gekennzeichnet, dass** durch das weitere Aufkonzentrieren eine konzentrierte Dünnschlempe mit einem Gehalt an Trockensubstanz von mehr als 12 Gew.-%, vorzugsweise 18 Gew.-% oder höher gebildet wird.

4. Verfahren nach Anspruch 2 oder 3, **dadurch gekennzeichnet, dass** nach der Klärung ein Trocknen der konzentrierten Dünnschlempe unter Bildung eines proteinhaltigen Wertproduktes erfolgt.

5. Verfahren nach einem der vorhergehenden Ansprüche, **dadurch gekennzeichnet, dass** der erste Teilstrom (TS1) nach der Strömungsteilung zumindest dreimal, vorzugsweise zumindest sechsmal größer ist als der zweite Teilstrom (TS2).

6. Verfahren nach einem der vorhergehenden Ansprüche, **dadurch gekennzeichnet, dass** die in den Arbeitsbehälter zugeführte Dünnschlempe (TS) durch Verarbeitung von Getreideschlempe (WS) in einem Dekanter (1) bereitgestellt wird.

7. Verfahren nach einem der vorhergehenden Ansprüche, **dadurch gekennzeichnet, dass** zusätzlich zur aufkonzentrierten Dünnschlempe ein Permeatstrom (P) aus der Filtrationsanlage (9) abgeführt wird, welcher einer anaeroben Abwasserreinigung zuführbar ist oder als Prozesswasser wieder verwendet werden kann.

8. Verfahren nach einem der vorhergehenden Ansprüche, **dadurch gekennzeichnet, dass** die Strömungsteilung nach dem Aufkonzentrieren von Dünnschlempe in einer Filtrationsanlage (9) in Abhängigkeit vom Trockensubstanzgehalt der im Arbeitsbehälter (3) befindlichen Dünnschlempe erfolgt.

9. Verfahren nach einem der Ansprüche 1-7, **dadurch gekennzeichnet, dass** die Strömungsregelung nach dem Aufkonzentrieren von Dünnschlempe in einer Filtrationsanlage (9) anhand der Durchflussrate geregelt wird.

10. Vorrichtung zur Herstellung eines proteinhaltigen Wertproduktes aus Dünnschlempe, mit einem Arbeitsbehälter (3), einer Filtrationsanlage (9), einer Zentrifuge, vorzugsweise einem Separator (16), und einer Trocknungsvorrichtung, **dadurch gekennzeichnet, dass**
die Filtrationsanlage (9) mit dem Arbeitsbehälter (3) über eine Rückführung (17) für einen ersten Teilstrom (TS1) der konzentrierten Dünnschlempe verbunden ist.

11. Vorrichtung nach Anspruch 10, **dadurch gekennzeichnet, dass** die Filtrationsanlage (9) eine Ultrafiltrationsanlage ist.

12. Vorrichtung nach Anspruch 10 oder 11, **dadurch gekennzeichnet, dass** die Filtrationsanlage (9) Mittel zur Strömungsteilung (15) der konzentrierten Dünnschlempe in einen ersten Teilstrom (TS1) und einen zweiten Teilstrom (TS2) aufweist.

13. Vorrichtung nach Anspruch 12, **dadurch gekennzeichnet, dass** die Mittel zur Strömungsteilung (15) in Abhängigkeit von der Durchflussrate geregelt werden.

14. Vorrichtung nach einem der vorhergehenden Ansprüche 10 bis 13,
**dadurch gekennzeichnet, dass** die Zentrifuge (16) mit dem Arbeitsbehälter (3) über eine Rückführung (18) verbunden ist, zum Rückführen der geklärten Zentrifugatphase in den Arbeitsbehälter (3).

## Claims

1. Method for processing thin stillage (TS), **characterized by** the following steps:
a) feeding of thin stillage (TS) into a working vessel (3);
b) concentrating thin stillage (TS) in a filtration unit (9);
c) wherein, after the concentration of the thin stillage in the filtration unit (9), and a flow splitting is done of the concentrated thin stillage into at least the first substream (TS1) and a second substream (TS2), and
d) returning the first substream (TS1) of concentrated thin stillage to the thin stillage (TS) contained in the working vessel (3) in order to adjust the amount of dry matter.

2. Method according to claim 1, **characterized in that** after the flow splitting, at least the second substream (TS2) of the concentrated thin stillage is clarified by a centrifuge, preferably by a separator (16), wherein the thin stillage is further concentrated.

3. Method according to Claim 2, **characterized in that** a concentrated thin stillage with a dry matter amount of more than 12 wt.%, preferably 18 wt. % or higher is formed by further concentrating.

4. Method according to Claim 2 or 3, **characterized in that** after clarification, the concentrated thin stillage is dried, forming a product of value containing protein.

5. Method according to one of the preceding claims, **characterized in that** the first substream (TS1) following the flow splitting is at least three times, preferably at least six times greater than the second substream (TS2).

6. Method according to one of the preceding claims, **characterized in that** the thin stillage (TS) fed to the working vessel is prepared by processing grain stillage (WS) in a decanter (1).

7. Method according to one of the preceding claims, **characterized in that** in addition to the concentrated thin stillage a permeate stream (P) is discharged from the filtration unit (9), the permeate stream being routable to an anaerobic wastewater treatment facility or being reusable as process water.

8. Method according to one of the preceding claims, **characterized in that** the flow splitting is done after the concentrating of thin stillage in a filtration unit (9) as a function of the dry matter content of the thin stillage located in the working vessel (3).

9. Method according to one of Claims 1-7, **characterized in that** flow control is regulated by way of the flow rate after concentration of thin stillage in a filtration unit (9).

10. Device for producing a valuable product containing protein from thin stillage, comprising a working vessel (3), a filtration unit (9), a centrifuge, preferably a separator (16), and a dryer,
**characterized in that**
the filtration unit (9) is connected to the working vessel (3) by way of a return line (17) for a first substream (TS1) of the concentrated thin stillage.

11. Device according to Claim 10, **characterized in that** the filtration unit (9) is an ultrafiltration unit.

12. Device according to Claim 10 or 11, **characterized in that** the filtration unit (9) comprises means for flow splitting (15) the concentrated stillage into a first substream (TS1) and a second substream (TS2).

13. Device according to Claim 12, **characterized in that** the means for flow splitting (15) is regulated as a function of the flow rate.

14. Device according to one of the preceding Claims 10 to 13, **characterized in that** the centrifuge (16) is connected to the working vessel (3) by way of a return line (18) for returning the clarified centrifugate phase to the working vessel (3).

## Revendications

1. Procédé de traitement de résidus solubles de distillation (TS), **caractérisé par** les étapes suivantes :
a) acheminement de résidus solubles de distillation (TS) dans un récipient de travail (3) ;
b) concentration de résidus solubles de distillation (TS) dans une installation de filtrage (9) ;
c) dans lequel après la concentration de résidus solubles de distillation dans une installation de filtrage (9), une division du courant de résidus solubles de distillation concentrés s'effectue en au moins un premier courant partiel (TS1) et en un deuxième courant partiel (TS2), et
d) recyclage du premier courant partiel (TS1) de résidus solubles de distillation concentrés dans le résidu soluble de distillation (TS) se trouvant dans le récipient de travail (3) pour l'ajustement de la teneur en substance sèche.

2. Procédé selon la revendication 1, **caractérisé en ce que**, après la division du courant, une clarification d'au moins le deuxième courant partiel (TS2) du résidu soluble de distillation concentré s'effectue dans une centrifugeuse, de préférence dans un séparateur (16), dans lequel s'effectue une autre concentration du résidu soluble de distillation.

3. Procédé selon la revendication 2, **caractérisé en ce que**, par le biais de l'autre concentration, on obtient un résidu soluble de distillation concentré présentant une teneur en substance sèche supérieure à 12 % en poids, de préférence, de 18 % ou plus de 18 % en poids.

4. Procédé selon la revendication 2 ou 3, **caractérisé en ce qu'**après la clarification s'effectue un séchage du résidu soluble de distillation en formant un produit de valeur contenant des protéines.

5. Procédé selon l'une des revendications précédentes, **caractérisé en ce que** le premier courant partiel (TS1) après la division du courant est au moins trois fois plus important, de préférence au moins six fois plus important que le deuxième courant partiel (TS2).

6. Procédé selon l'une des revendications précédentes, **caractérisé en ce que** le résidu soluble de distillation (TS) acheminé dans le récipient de travail est mis à disposition par traitement de produits de distillation de céréales (WS) dans un décanteur (1).

7. Procédé selon l'une des revendications précédentes, **caractérisé en ce que**, en plus du résidu soluble de distillation concentré, un courant de perméat (P) est évacué de l'installation de filtrage (9), ledit courant de perméat (P) peut être acheminé à une épuration anaérobie des eaux usées ou peut être réutilisé comme eau de processus.

8. Procédé selon l'une des revendications précédentes, **caractérisé en ce que** la division du courant s'effectue après la concentration du résidu soluble de distillation dans une installation de filtrage (9) en fonction de la teneur en substance sèche du résidu soluble de distillation se trouvant dans le récipient de travail (3).

9. Procédé selon l'une des revendications 1 à 7, **caractérisé en ce que** le réglage du courant est réalisé après la concentration du résidu soluble de distillation dans une installation de filtrage (9) sur la base du débit.

10. Dispositif de production d'un produit de valeur contenant des protéines à partir d'un résidu soluble de distillation avec un récipient de travail (3), une installation de filtrage (9), une centrifugeuse, de préférence, un séparateur (16) et un dispositif de séchage, **caractérisé en ce que**
l'installation de filtrage (9) est reliée au récipient de travail (3) par une conduite de retour (17) pour un premier courant partiel (TS1) du résidu soluble de distillation concentré

11. Dispositif selon la revendication 10, **caractérisé en ce que** l'installation de filtrage (9) est une installation d'ultrafiltration.

12. Dispositif selon la revendication 10 ou 11, **caractérisé en ce que** l'installation de filtrage (9) présente des moyens de division du courant (15) du résidu soluble de distillation concentré en un premier courant partiel (TS1) et un deuxième courant partiel (TS2).

13. Dispositif selon la revendication 12, **caractérisé en ce que** les moyens de division du courant (15) sont réglés en fonction du débit.

14. Dispositif selon l'une des revendications 10 à 13 précédentes, **caractérisé en ce que** la centrifugeuse (16) est reliée au récipient de travail (3) par une conduite de retour (18) pour le recyclage de la phase de centrifugeat clarifiée dans le récipient de travail (3).
